# EUROPEAN PATENT APPLICATION

(11) **EP 1 327 392 A1**
(43) Date of publication of application: **16.07.2003**
(21) Application number: 01972112.5
(22) Date of filing: 04.10.2001
(51) Int. Cl.: A23L 3/015, B01J 3/02

(54) **MACHINE FOR HIGH PRESSURE PROCESSING OF PRODUCTS**

(30) Priority: 06.10.2000 ES 200002419
(71) Applicant: Amahe, S.A., 09001 Burgos (ES)
(72) Inventor: HERNANDO SAIZ, Andrés, Polig. Villalonquéjar, 09001 Burgos (ES)
(74) Representative: Gil Vega, Victor
(86) International application number: ES0100368
(87) International publication number: WO02028205

(57) **Abstract**

Said machine is intended for the food industry for the sterilization of products (88) by applying high pressures at 4,000 bars. The machine includes a chamber (1) which adopts a horizontal position and has input/output mouths (5) on both ends. The plugs (6) for closing said chamber (1) are placed on a fixed yoke (4) in relation to which the chamber (81) can move transversally in the opening position so that the chamber can be inserted in the transport line (7) of the products (8) to be processed in such a way that the loading of a batch of products to be processed and the unloading of products already processed take place simultaneously once the chamber (1) has been inserted into said line (7). Transversal displacement of the chamber (1) into the yoke (4) makes it possible to close and pressurize the chamber.

## Description

### ABSTRACT OF THE INVENTION

The present invention involves a type of machine for treating certain products, generally food ones, by means of high pressure, with the purpose to extend the shelf life of them.

The above mentioned machine is especially suited for the food industry environment.

### BACKGROUND OF THE INVENTION

There is an increasing tendency to treat food products with high pressure, about 4000 bar or even more. To achieve and withstand those high pressures high quality stainless steels are required, which results in a very high cost.

These big investments are only returnable if the machine is able to process enough products by time unit.

So far, machines which are presently known use a chamber to introduce the product to be pressurized, in a vertical position, so the chamber is filled or emptied with the product through the same orifice, resulting in a lost of time in each cycle. The cycle time is defined as the amount of time from which the product is introduced into the chamber to the introduction of the next batch of products to be treated by pressure. That is the reason why, with the vertical design, it is not possible to enter a new batch until the extraction of the previous one is finished.

On the other hand, with the machines that are known so far a temperature control system is used in parallel, which in the case of a double wall chamber, made of an external vessel and an internal liner, consist of two complementary channels, which are grooved in the inner surface of the vessel and in the outer surface of the liner. As a result of these set ups, both channels form a conduction, which is used to circulate the fluid of thermic control. In practice, these channels not only result in a very high cost, but also are really sensitive to crack generation and consequently the lifetime is shorter.

### DESCRIPTION OF THE INVENTION

The machine that the invention proposes solves in a satisfactory way the above mentioned problem in all the related aspects.

In order to achieve that, the chamber set up of the machine is horizontal and it has one opening in each end, so the products load and unload can be performed simultaneously. To be more specific, the machine can be unloaded from one opening and, at the same time, loaded through the other opening. This means that the cycle time can be reduced significantly in contrast with the traditional method.

Additionally, and according to another characteristic of the invention, the chamber is disposed in between a fixed yoke, across which the chamber can be moved. Then the mentioned chamber is aligned with the product line at the same time that the load and the unload are performed, avoiding the final product to be mixed by mistake with the untreated product. The plugs, which close the chamber before it is pressurized, are disposed in the mentioned yoke.

According to another characteristic of the invention, the machine is provided with a fast load/unload system at low pressure, consisting of a ring coupled to an end of the chamber with a low pressure conduit connecting the interior and the exterior of the chamber.

In this sense it has also been foreseen to use the movement of the plugs at different depths in their way to the chamber, allowing or not the obstruction of the low pressure conduit of the ring. For filling/emptying the chamber at low pressure the plugs will be situated in the no obstruction position of this conduit and for the filling/emptying at high pressure the plugs will be in the obstruction position.

Finally, according to another characteristic of the invention, the double wall that forms the chamber is made of an interior liner and an exterior vessel in between which a helical winded strip is established, so an also helical channel is defined between the winding of the strip. This channel is used to circulate the thermic control liquid, avoiding the presence of edges in the wall of the chamber that would facilitate the appearance of cracks.

### DESCRIPTION OF THE DRAWINGS

In order to complete the description of the invention and with the target to better understand its characteristics, according to a preferred execution of the same, a set of drawings is enclosed as a part of the description, with an illustrative and non-limiting purpose, showed in the following:
Figure 1.- Shows a perspective schematic view of a machine for food processing, built according to the object of the present invention, in a position that represents the chamber transversally displaced with respect to the fixed yoke and aligned with the product transport line, for product load/unload.
Figure 2.- Shows a perspective partial detail in one fourth section of the end of the chamber shown in the former figure, in which is placed the low pressure input/output ring.
Figure 3.- Shows a perspective partial schematic view of the liner that is shown in the former figure with the additional helical strip that determines the liquid circulation conduit for the temperature control, in which a zoom allows a better view of the liquid conduit.

### PREFERRED EMBODIMENT OF THE INVENTION

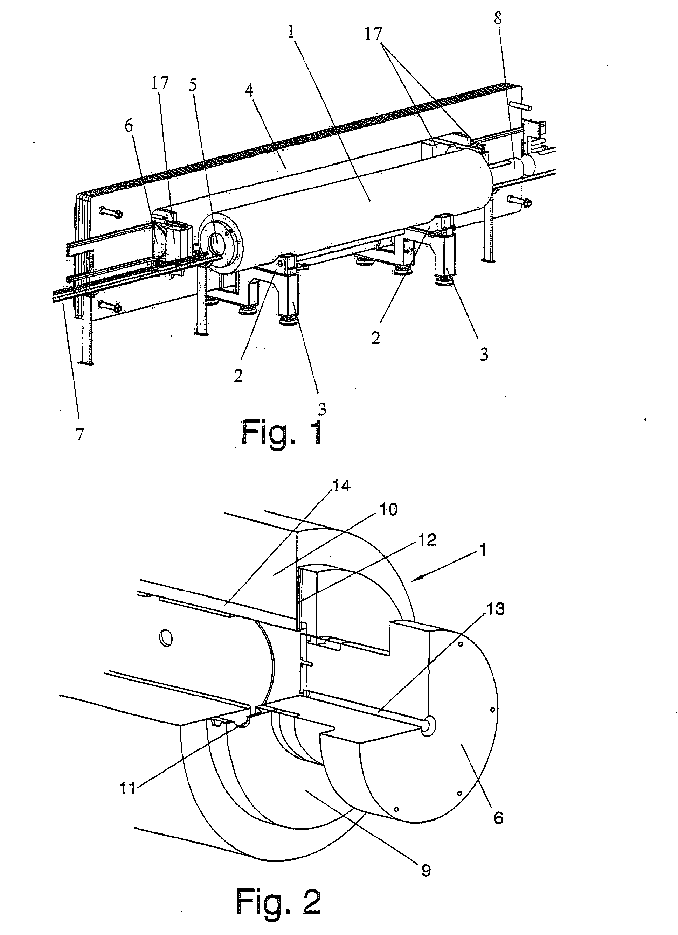

According to the figures mentioned before, especially figure 1, it can be observed how the machine is provided with a chamber (1), which is horizontally disposed, held over two supports (2) that transversally glide over the bench (3) with respect to the axe chamber (1), thus allowing the transversal displacement of the chamber with respect to the yoke (4), which stands still. Before the pressurization phase, the chamber (1) is displaced to the inner part of the yoke (4) and the closure of the openings (5) is performed through the plugs (6) foreseen in the yoke (4), with the later introduction of the wedge (17) in between the plugs (6) and the yoke (4) in order to transmit to the latter the mechanical efforts that tends to open the plugs (6) as a result of the high pressure reached in the chamber (1). After the pressurization phase, the chamber (1) is transversally displaced from the inside part of the yoke (4) to the outside part in order to be aligned with the product transport line (7), as shown in figure 1, so that the products (8) to be treated come into the chamber (1) directly from one of the extremities at the same time that the products already treated come out from the opening (5) of the chamber (1) in the other extremity, in the normal flow through the transport product line (7).

This implies that the load/unload operations can be performed simultaneously with the consequent of saving time that results from minimising the "cycle time", i.e. the elapsed time from the introduction of a product batch (8) into the chamber (1) until the introduction of the next product batch. This operation mode also allows the obtaining of a continuous product flow and the complete traceability during the treatment, avoiding the exchange of untreated and processed product by mistake. Consequently, the mentioned horizontal disposal of the chamber (1) enhances the product's quality management, by integrating the machine into a continuous flow line in which the probability of a non-treated product crosses the machine decreases.

The reduction of the machine's cycle time is also achieved by means of a new fast chamber's filling/emptying system at low pressure. This system, which can be identified in figure 2, is made by a ring (9) screwed to the vessel (10) or exterior wall of the chamber (1). This ring (9) also has a conduit (11) that communicates the chamber (1) with the exterior, and has also an air output orifice (12), to prevent air bubbles forming during the filling of the chamber (1).

Being more specific, when the plug (6) is brought to the position that figure 2 represents, the liquid is introduced into the chamber (1) throughout the conduit (11) until all the air from the chamber has escaped through the orifice (12). After that, the plug (6) is displaced until the chamber (1) is totally closed, and the liquid exits, as a result of the volume reduction, through the high pressure orifice (13). Finally, the chamber is pressurized to the established high pressure by means of the previously mentioned high pressure conduit (13).

It is clear that the emptying of the chamber is done in the reverse order of the one described here.

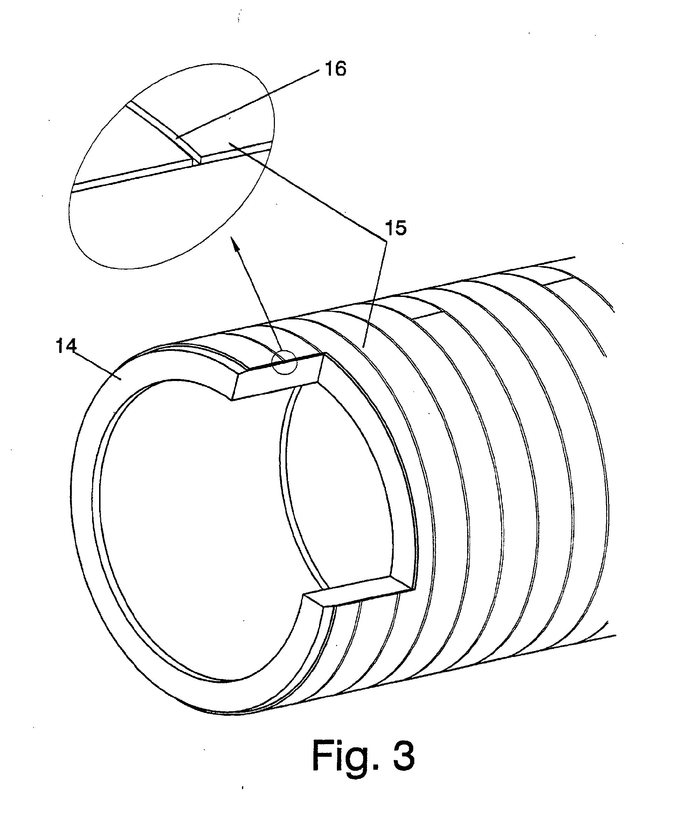

Finally to control the temperature in the chamber (1) the circulation of water is used, as is conventionally done, through the double wall that constitutes the chamber. To be more precise, between the vessel (10) and the liner (14) it has been foreseen a helical strip (15), as it can be especially seen in figure 3, that surrounds the liner (14), in between the spires an empty channel (16) is defined. This channel will be large enough to allow circulation of the liquid, thus to perform the thermic control. This is the reason why the thickness of the strip and the distance between spires must be adequate, so when mounting the vessel (10) over the unit formed by the liner (14) and the strip (15) showed in figure 3, a helical channel (16) for thermic control is achieved in the wall of the chamber (1).

## Claims

1. Machine for treatment of products by high pressure, for example food products, which are subjected to cold pasteurization by applying pressures to them around 4000 bar or even greater in the core of a pressurization chamber (1), **characterized in that** the chamber (1) takes a horizontal position and is provided with two openings (5) for loading/unloading product (8) to be treated, having been foreseen the closing plugs (6) of the chamber (1) to be over the fixed yoke (4) and, also the wedges system (17) to be put in between the plugs (6) and the yoke (4), thus allowing to transmit the mechanical efforts that tends to open the plugs (6) during the pressurization, being the chamber transversally movable respect to the yoke (4), across a lower bench (3), in order to align it with the transport line (7) for the products (8) to be treated, so that when a batch is coming into one opening (5) of the chamber (1), simultaneously the previous one with products already treated is coming out from the other end (5).

2. Machine for treatment of products by high pressure, according to claim 1, **characterized by** a fast filling/emptying system, at low pressure, consisting of a ring (9) next to the chamber (1), which has a low pressure conduit (11) that connects the inside and the outside zones of the chamber (1).

3. Machine for treatment of products by high pressure, according to claim 2, **characterized in that** the plug (6) located accordingly with the end of the chamber (1) in which is located the ring (9), is able to move respectively to the chamber (1) to different depths of penetration in the same, in order to close or not the path of the low pressure conduit (11), being placed in the no-obstruction position for the low pressure filling/emptying of the chamber (1) through the conduit (11) and in the obstruction position for the filling/emptying of the chamber at high pressure through the proper high pressure conduit (13) preventing the low pressure conduit (11) from being under high pressure.

4. Machine for treatment of products by high pressure, according to any of the previous claims, **characterized by** a temperature control system in the chamber (1), based on a thermic liquid channel (16) established in between the double wall of the chamber (1), composed of the vessel (10) and the liner (14), and defined by a strip (15) which is helically winded onto the liner (14), forming a helical channel (16) in the junction with the other complementary part of the chamber (1), the vessel (10), being the thickness of the above mentioned strip (15) and the distance in between the spires the adequate for the flow of the thermic control liquid foreseen throughout this helical conduit (16).

5. Machine for treatment of products by high pressure, according to claim 4, **characterized in that** the chamber (1) is formed by multiple walls and is provided with the same temperature control system established in between any of the different walls that constitutes it.
